(19) Europäisches Patentamt
European Patent Office
Office européen des brevets

(11) Veröffentlichungsnummer: **0 004 920**
**B1**

(12) # EUROPÄISCHE PATENTSCHRIFT

(45) Veröffentlichungstag der Patentschrift: **05.08.81**

(51) Int. Cl.³: **C 07 D 209/88,** A 61 K 31/40, C 07 D 401/12, C 07 D 405/12

(21) Anmeldenummer: **79101063.0**

(22) Anmeldetag: **07.04.79**

(54) **Carbazolyl-(4)-oxy-propanolamin-Derivate, Verfahren zu ihrer Herstellung und diese Verbindungen enthaltende Arzneimittel.**

(30) Priorität: **13.04.78 DE 2815926**

(43) Veröffentlichungstag der Anmeldung: **31.10.79 Patentblatt 79/22**

(45) Bekanntmachung des Hinweises auf die Patenterteilung: **05.08.81 Patentblatt 81/31**

(84) Benannte Vertragsstaaten: **BE CH DE FR GB IT LU NL SE**

(56) Entgegenhaltungen:
**AT-B-336 176**
**DE-C-2 240 599**
**DE-A-2 424 523**
**DE-A-2 454 406**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Wiedemann, Fritz, Dr., Weinheimer Strasse 82, D-6940 Weinheim-Lützelsachsen (DE)**
Erfinder: **Kampe, Wolfgang, Dr., Zedernstrasse 49, D-6805 Heddesheim (DE)**
Erfinder: **Thiel, Max, Dr., S 6, 35, D-6800 Mannheim 1 (DE)**
Erfinder: **Sponer, Gisbert, Dr., Tilsiterstrasse 30, D-6944 Hemsbach (DE)**
Erfinder: **Roesch, Egon, Dr., Am Oberen Luisenpark 22, D-6800 Mannheim 1 (DE)**
Erfinder: **Dietmann, Karl, Prof. Dr., Eisenacher Weg 75, D-6800 Mannheim-Vogelstang (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Carbazolyl-(4)-oxy-propanolamin-Derivate, Verfahren zu ihrer Herstellung und diese Verbindungen enthaltende Arzneimittel

Die Erfindung betrifft Carbazolyl-(4)-oxy-propanolamin-Derivate, Verfahren zu ihrer Herstellung und diese Verbindungen enthaltende Arzneimittel.

Diese Verbindungen sowie ihre pharmakologisch unbedenklichen Salze zeigen im pharmakologischen Test vasodilatierende und β-rezeptorenblockierende Wirkungen und eignen sich daher zur Behandlung und Prophylaxe bei Kreislauf- und Herzerkrankungen, wie z.B. Hypertension und Angina pectoris.

Aus der DE-PS 2240599 sind bereits Carbazol-Derivate bekannt, welche die Aktivität der β-Rezeptoren des Sympathicus blockieren.

Die Erfindung betrifft Carbazolyl-(4)-oxy-propanolamin-Derivate der Formel I, ihre Salze mit physiologisch verträglichen Säuren, Verfahren zu ihrer Herstellung und die Verwendung zur Herstellung von Arzneimitteln.

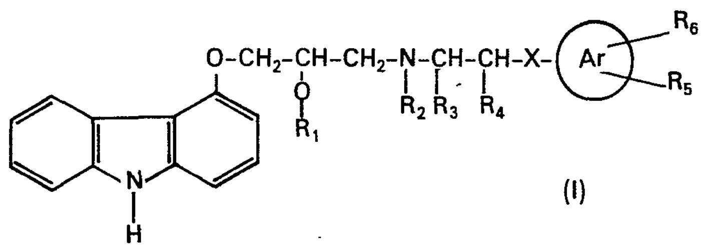

(I)

In der Formel I bedeuten:

$R_1$ Wasserstoff, eine niedere Alkanoylgruppe oder eine Benzoyl- oder Naphthoylgruppe,

$R_2$ Wasserstoff, eine niedere Alkylgruppe oder eine Benzyl-, Phenylethyl- oder Phenylpropylgruppe,

$R_3$ Wasserstoff oder eine niedere Alkylgruppe,

$R_4$ Wasserstoff, eine niedere Alkylgruppe,

X einen Valenzstrich, eine $-CH_2$-Gruppe, ein Sauerstoff, oder ein Schwefelatom,

Ar einen Phenyl-, Naphthyl-, Indanyl-, Tetrahydronaphthyl- oder Pyridyl-Rest und

$R_5$ und $R_6$, welche gleich oder verschieden sein können, jeweils Wasserstoff, Halogen, eine niedere Alkylgruppe, eine Aminocarbonylgruppe, eine Hydroxygruppe, eine niedere Alkoxygruppe, eine Benzyloxygruppe, eine niedere Alkylmercaptogruppe, eine niedere Alkylsulfinylgruppe, eine niedere Alkylsulfonylgruppe oder auch gemeinsam eine Methylendioxygruppe bedeuten, wobei für den Fall, das X ein Sauerstoffatom darstellt, $R_4$ und $R_5$ gemeinsam auch die Gruppe $-CH_2-O-$ bedeuten kann.

Die niederen Alkylreste der Substituenten $R_2$, $R_3$, $R_4$, $R_5$ und $R_6$ enthalten 1-6, vorzugsweise 1-4 Kohlenstoffatome und können geradkettig oder verzweigt sein. Als bevorzugt zu nennen sind der Methyl-, Ethyl-, Isopropyl-, t.-Butyl- und n-Butyl-Rest.

Niedere Alkanoyl-Gruppen sind Gruppen mit 1-6 Kohlenstoffatomen, insbesondere die Formyl-, Acetyl-, Propionyl- und Pivaloylgruppe.

Unter Halogen versteht man Fluor, Chlor und Brom.

Eine niedere Alkoxy-, Alkylmercapto-, Alkylsulfinyl- oder Alkylsulfonylgruppe kann 1-6, vorzugsweise 1-4 Kohlenstoffatome enthalten. Bevorzugt sind die Methoxy- und die Ethoxygruppe, die Methylmercapto-, die Methylsulfinyl- und die Methylsulfonylgrupe.

Als Beispiel für einen Rest, bei dem $R_4$ und $R_5$ zusammen eine $-CH_2-O$-Gruppe bilden, wenn X = Sauerstoff bedeutet, ist der 1,4-Benzodioxanyl-(2)-methyl-Rest zu nennen.

Die erfindungsgemässen Verbindungen sowie ihre pharmakologisch unbedenklichen Salze weisen im Vergleich zu den bisher bekannten Substanzen mit ähnlicher Konstitution und Wirkung neben einer ausgeprägten β-rezeptorenblockierenden Wirkung auch gute vasodilatierende Eigenschaften auf.

Zum Beweise hierfür wurde im pharmakologischen Test als Vertreter der erfindungsgemässen Verbindungen die Verbindung des Beispiels 1, 1-[Carbazolyl-(4)-oxy]-3-[2-(2-methoxyphenoxy)-ethylamino]-propanol-(2), mit dem 4-(3-Isopropylamino-2-hydroxy-propoxy)-carbazol-hydrochlorid aus der DE-PS 2240599 verglichen.

Als Mass für die Vasodilatation wurde an narkotisierten Kaninchen der $DE_{-30\ mm\ Hg}$-Wert bestimmt, d.h. die Dosis der pharmakologisch aktiven Verbindung, die den CSE-Reflex (temporäre Erhöhung des Blutdruckes durch Occludieren der Halsschlagadern für 2 Minuten) um 20 mm Hg abschwächt. Als Mass für die β-Rezeptoren-Blockade diente die an wachen Kaninchen ermittelte $DE_{250}$, d.h. die Dosis des β-Blockers, bei der die Herzfrequenz nach Injektion von Isoprenalin nur noch auf 250 Schläge/min ansteigt.

Die Auswertung dieser Versuche ergab, dass für die ausgewählte erfindungsgemässe Verbindung der Quotient aus dem $DE_{-30\ mm\ Hg}$- und $DE_{250}$-Wert bei 1 liegt, d.h. β-Rezeptoren-Blockade und Vasodilatation liegen in gleicher Grössenordnung. Für die aus der DE-PS 2240599 bekannte Vergleichssubstanz wurde ein Quotient von über 100 gefunden, d.h. diese Verbindung weist praktisch nur β-blockierende Eigenschaften auf; die durch diese Substanz bewirkte Vasodilatation ist vernachlässigbar gering.

Anhand einer geeigneten Auswahl von Substanzen konnte gezeigt werden, dass auch die anderen erfindungsgemässen Verbindungen β-blockierende und vasodilatierende Eigenschaften in vergleichbarer Grössenordnung besitzen.

Die Herstellung der erfindungsgemässen Verbindungen ist dadurch gekennzeichnet, dass man in an sich bekannter Weise

a) eine Verbindung der Formel II

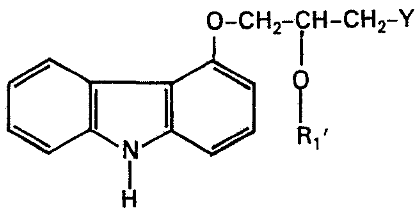

(II)

in welcher Y eine reaktive Gruppe darstellt und $R_1'$ die für $R_1$ angegebene Bedeutung hat oder Y und $R_1'$ zusammen einen Valenzstrich bedeuten, mit einer Verbindung der Formel III

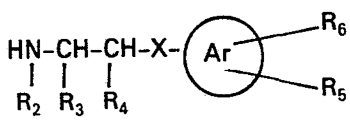

(III)

in welcher $R_2$, $R_3$, $R_4$, X, Ar, $R_5$ und $R_6$ die angegebene Bedeutung haben,
umsetzt oder

b) eine Verbindung der Formel IV

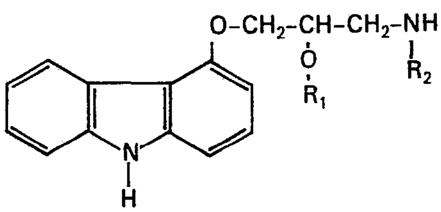

(IV)

in welcher $R_1$ und $R_2$ die angegebene Bedeutung haben,
mit einer Verbindung der Formel V

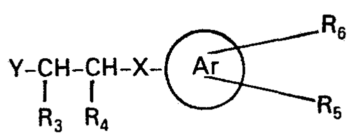

(V)

in welcher Y, $R_3$, $R_4$, X, Ar, $R_5$ und $R_6$ die angegebene Bedeutung haben,
umsetzt oder

c) ein Gemisch aus einer Verbindung der Formel IV und einer Verbindung der Formel VI

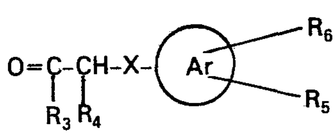

(VI)

in welcher $R_3$, $R_4$, X, Ar, $R_5$ und $R_6$ die angegebene Bedeutung haben,
reduziert oder

d) eine Verbindung der Formel IV mit einer Verbindung der Formel VII

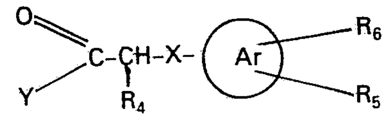

(VII)

in welcher Y, $R_4$, X, Ar, $R_5$ und $R_6$ die angegebene Bedeutung haben,
umsetzt und das so erhaltene Amid reduziert, worauf man gegebenenfalls die nach obigen Verfahren hergestellten Verbindungen der Formel I in andere Verbindungen der Formel I umwandelt sowie gewünschtenfalls die erhaltenen Verbindungen der Formel I in ihre pharmakologisch verträglichen Salze überführt.

Reaktive Gruppen Y der Verbindungen der Formel II, V und VII sind insbesondere Säurereste, z.B. von Halogenwasserstoffsäuren oder Sulfonsäuren.

Die erfindungsgemässen Verfahren a) und b) werden zweckmässig in einem unter den Reaktionsbedingungen inerten organischen Lösungsmittel, z.B. Toluol, Dioxan, Ethylenglykoldimethylether, Isopropanol oder Dimethylformamid, gegebenenfalls in Gegenwart eines säurebindenden Mittels durchgeführt. Die Umsetzungen der Epoxide der Formel II (Y und $R_1'$ zusammen ein Valenzstrich) mit den Aminen der Formel III kann aber auch nach Mischen der Reaktionskomponenten durch Stehenlassen bei Raumtemperatur oder durch Erhitzen bewirkt werden. Nach Verfahren c) wird ein Amin der Formel IV mit einer Carbonylverbindung der Formel VI in einem geeigneten Lösungsmittel (z.B. Methanol) in Gegenwart eines Katalysators (z.B. Raney-Nickel) hydriert.

Die Reduktion der nach Verfahren d) erhaltenen Amide erfolgt mittels komplexer Metallhydride, z.B. Lithiumaluminiumhydrid. Die Veresterung von Verbindungen der Formel I mit $R_1 = H$ kann durch Umsetzung mit einem Säurehalogenid oder Säureanhydrid, ggf. in Gegenwart eines säurebindenden Mittels (z.B. Pyridin, Triethylamin), die Entfernung einer evtl. vorhandenen Benzylschutzgruppe durch katalytische Hydrierung mit Edelmetallkatalysatoren erfolgen.

Die bei dem erfindungsgemässen Verfahren eingesetzten Ausgangsverbindungen sind in der Regel literaturbekannte Verbindungen. Neue Verbindungen werden im allgemeinen analog den für die Herstellung dieser bekannten Verbindungen beschriebenen Verfahren erhalten. So können die Amine der Formel III vorzugsweise durch Umsetzung von Halogenalkyl-nitrilen mit entsprechenden Phenolen, Naphtholen oder Aryl-Verbindungen (z.B. Chloracetonitril und Phenol) und anschliessender Hydrierung in Gegenwart von Ammoniak hergestellt werden.

Die Amine der Formel IV können aus dem bekannten 4-(2,3-Epoxypropoxy)-carbazol (vergl. DG-PS 22 40 599) durch Umsetzung mit flüssigem Ammoniak erhalten werden.

Reaktive Verbindungen der Formel V z.B. p-Toluolsulfonsäureester werden in der Regel aus den entsprechenden Phenolen, Naphtholen oder Aryl-Verbindungen durch Umsetzung mit Halogen-alkoholen und anschliessender Veresterung mit p-Toluol-sulfonsäure hergestellt.

Die Carbonyl-Verbindungen der Formel VI und Säurechloride der Formel VII werden aus entsprechenden Phenolen, Naphtholen und Arylverbindungen über Umsetzungen mit geeigneten Halogenalkylverbindungen erhalten.

Eine nachträgliche Umwandlung einer Verbindung der Formel I in eine andere Verbindung der Formel I kann beispielsweise durch Oxidation geschehen, z.B. Überführung einer Alkylmercaptogruppe in eine Alkylsulfinyl- oder Alkylsulfonylgruppe. Ferner können Hydroxygruppen nach bekannten Methoden verethert oder verestert werden, bzw. umgekehrt Ester- und Ethergruppen in Hydroxygruppen überführt werden.

Zur Überführung der Verbindungen der Formel I in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Phosphorsäure, Schwefelsäure, Essigsäure, Citronensäure, Maleinsäure, Benzoesäure um.

Die erfindungsgemässen Verbindungen der Formel I können aus ihren racemischen Mischungen nach an sich bekannten Methoden über die diastereomeren Salze in die optisch aktiven Formen aufgetrennt werden. Zur Racematspaltung können z.B. Weinsäure, Äpfelsäure, Camphersäure, Camphersulfonsäure verwendet werden.

Zur Herstellung von Arzneimitteln werden die Verbindungen der Formel I in an sich bekannter Weise mit geeigneten pharmazeutischen Trägerstoffen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die erfindungsgemässen Verbindungen der Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nicht-toxische Salze), hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole), für orale Applikation geeignete Zubereitungen können gewünschtenfalls Geschmacks- und Süsstoffe enthalten.

Die Erfindung wird durch die folgenden Beispiele näher erläutert:

**Beispiel 1**

1-[Carbazolyl-(4)-oxy]-3-[2-(2-methoxyphenyl)-ethylamino]-propanol-(2)

6,0 g 4-(2,3-Epoxypropoxy)-carbazol und 7,6 g 2-(2-Methoxyphenyl)-ethylamin werden 20 Stunden bei 70°C gerührt. Man reibt mit Ether an, saugt ab und kristallisiert aus Essigester unter Verwendung von Aktivkohle und Bleicherde um. Ausbeute: 6,0 g (61% d.Th.) farblose Kristalle, Fp. 135–136°C.

In analoger Weise erhält man:

a) 1-[Carbazolyl-(4)-oxy]-3-[2-(3,4-dimethoxyphenyl)-ethylamino]-propanol-(2)
42% d.Th., Fp. 129–130°C, essigsaures Salz Fp. 180–183°C, aus 4-(2,3-Epoxypropoxy)-carbazol und 2-(3,4-Dimethoxyphenyl)-ethylamin.

b) 1-[Carbazolyl-(4)-oxy]-3-[2-(2-pyridyl)-ethylamino]-propanol-(2)
32% d.Th., Fp. 105–107°C,
aus 4-(2,3-Epoxypropoxy)-carbazol und 2-(2-Pyridyl)-ethylamin.

c) 1-[Carbazolyl-(4)-oxy]-3-[2-(4-pyridyl)-ethylamino]-propanol-(2)
24% d.Th., Fp. 86–88°C,
aus 4-(2,3-Epoxypropoxy)-carbazol und 2-(4-Pyridyl)-ethylamin.

d) 1-[Carbazolyl-(4)-oxy]-3-(3-phenylpropylamino)-propanol-(2)
30% d.Th., bernsteinsaures Salz Fp. 98–99°C,
aus 4-(2,3-Epoxypropoxy)-carbazol und 3-Phenylpropylamin.

e) 1-[Carbazolyl-(4)-oxy]-3-[4-phenyl-butyl-(2)-amino]-propanol-(2)
13% d.Th., Fp. 124–125°C,
aus 4-(2,3-Epoxypropoxy)-carbazol und 4-Phenylbutyl-(2)-amin.

**Beispiel 2**

1-[Carbazolyl-(4)-oxy]-3-[2-(2-methoxyphenoxy)-ethylamino]-propanol-(2)

22,6 g 4-(2,3-Epoxypropoxy)-carbazol und 17,4 g 2-(2-Methoxyphenoxy)-ethylamin 75 ml Ethylenglykoldimethylether werden 25 Stunden bei 50°C gerührt. Man bringt das Gemisch am Rotavapor zur Trockene, reibt mit Ether an und kristallisiert aus Essigester unter Verwendung von Aktivkohle um.

Ausbeute: 15,1 g (39% d.Th.) farblose Kristalle, Fp. 114–115°C.

In analoger Weise erhält man:

a) 1-[Carbazolyl-(4)-oxy]-3-(2-phenoxy-ethylamino)-propanol-(2)
32% d.Th., Fp.105–107°C,
aus 4-(2,3-Epoxypropoxy)-carbazol und 2-Phenoxy-ethylamin.

b) 1-[Carbazolyl-(4)-oxy]-3-[1-phenoxy-propyl-(2)-amino]-propanol-(2)
31% d.Th., Hydrochlorid Fp. 116–119°C,
aus 4-(2,3-Epoxypropoxy)-carbazol und 1-Phenoxy-propyl-(2)-amin.

c) 1-[Carbazolyl-(4)-oxy]-3-[1,4-benzodioxanyl-(2)-methylamino]-propanol-(2)
28% d.Th., Fp. 129–131°C,
aus 4-(2,3-Epoxypropoxy)-carbazol und 2-(Aminomethyl)-1,4-benzodioxan.

d) 1-[Carbazolyl-(4)-oxy]-3-[2-(4-carbamoyl-

phenoxy)-ethylamino]-propanol-(2)
13% d.Th., Fp. 120–122 °C,
aus 4-(2,3-Epoxypropoxy)-carbazol und 2-(4-Carbamoylphenoxy)-ethylamin.

Beispiel 3

1-[Carbazolyl-(4)-oxy]-3-[2-(2-ethoxyphenoxy)-ethylamino]-propanol-(2)

6,0 g 4-(2,3-Epoxypropoxy)-carbazol und 9,1 g 2-(2-Ethoxyphenoxy)-ethylamin werden 20 Stunden bei 70 °C gerührt. Nach Erkalten rührt man mit Ether, saugt ab und kristallisiert den Rückstand aus Essigester unter Verwendung von Aktivkohle und Bleicherde um. Ausbeute: 4,4 g (42% d.Th.) farblose Kristalle, Fp. 127,5–128,5 °C.

In analoger Weise erhält man:

a) 1-[Carbazolyl-(4)-oxy]-3-[2-(4-fluorphenoxy)-ethylamino]-propanol-(2)
56% d.Th., Fp. 145–146 °C,
aus 4-(2,3-Epoxypropoxy)-carbazol und 2-(4-Fluorphenoxy)-ethylamin.

b) 1-[Carbazolyl-(4)-oxy]-3-[2-(4-tert.-butylphenoxy)-ethylamino]-propanol-(2)
51% d.Th., Fp. 127–128 °C,
aus 4-(2,3-Epoxypropoxy)-carbazol und 2-(4-tert.-Butylphenoxy)-ethylamin.

c) 1-[Carbazolyl-(4)-oxy]-3-[2-(2,3-dimethylphenoxy)-ethylamino]-propanol-(2)
51% d.Th., Fp. 128–129 °C,
aus 4-(2,3-Epoxypropoxy)-carbazol und 2-(2,3-Dimethylphenoxy)-ethylamin.

d) 1-[Carbazolyl-(4)-oxy]-3-[2-[indanyl-(5)-oxy]-ethylamino]-propanol-(2)
54% d.Th., Fp. 143–145 °C,
aus 4-(2,3-Epoxypropoxy)-carbazol und 2-[Indanyl-(5)-oxy]-ethylamin.

e) 1-[Carbazolyl-(4)-oxy]-3-[2-[naphthyl-(1)-oxy]-ethylamino]-propanol-(2)
64% d.Th., Fp. 116–119 °C,
aus 4-(2,3-Epoxypropoxy)-carbazol und 2-[Naphthyl-(1)-oxy]-ethylamin.

f) 1-[Carbazolyl-(4)-oxy]-3-[2-(3,4-methylendioxyphenoxy)-ethylamino]-propanol-(2)
32% d.Th., Fp. 142–143 °C,
aus 4-(2,3-Epoxypropoxy)-carbazol und 2-(3,4-Methylendioxyphenoxy)-ethylamin.

g) 1-[Carbazolyl-(4)-oxy]-3-[2-(2,6-dimethoxyphenoxy)-ethylamino]-propanol-(2)
65% d.Th., 136–138 °C,
aus 4-(2,3-Epoxypropoxy)-carbazol und 2-(2,6-Dimethoxyphenoxy)-ethylamin.

h) 1-[Carbazolyl-(4)-oxy]-3-[2-(2-methoxyphenoxy)-propylamino]-propanol-(2)
83% d.Th., Fp. 137–157 °C (rohes Gemisch der Diastereomeren), daraus durch zweimaliges Umkristallisieren aus Essigester: 22% d.Th., Fp. 173–175 °C,
aus 4-(2,3-Epoxypropoxy)-carbazol und 2-(2-Methoxyphenoxy)-propylamin.

i) 1-[Carbazolyl-(4)-oxy]-3-[2-(2-methylmercaptophenoxy)-ethylamino]-propanol-(2)
40% d.Th., Fp. 83–85 °C,
aus 4-(2,3-Epoxypropoxy)-carbazol und 2-(2-Methylmercaptophenoxy)-ethylamin.

k) 1-[Carbazolyl-(4)-oxy]-3-[2-(2-benzyloxyphenoxy)-ethylamino]-propanol-(2)
56% d.Th., Fp. 138–139 °C,
aus 4-(2,3-Epoxypropoxy)-carbazol und 2-(2-Benzyloxyphenoxy)-ethylamin.

Die in den Beispielen 3c, 3d, 3f, 3g und 3i verwendeten Ausgangsamine können durch literaturanaloge Umsetzungen über die entsprechenden Nitrile hergestellt werden.

2,3-Dimethylphenoxy-acetonitril

100 g 2,3-Dimethylphenol, 57 ml Chloracetonitril, 110 g Kaliumcarbonat und 2,0 g Kaliumiodid werden in 300 ml Methylethylketon 5 Stunden unter Rückfluss gerührt. Man saugt ab, engt ein, destilliert den Rückstand und erhält 88,0 g farbloses Öl, $Kp_{13}$ 137–142 °C.

Analog erhält man durch Umsetzungen von 5-Indanol, 3,4-Methylendioxyphenol bzw. 2-(Methylmercapto)-phenol mit Chloracetonitril:
Indanyl-(5)-oxy-acetonitril, $Kp_{14}$ 162–165 °C
3,4-Methylendioxyphenoxy-acetonitril, $Kp_{12}$ 170–175 °C
2-Methylmercaptophenoxy-acetonitril, Fp. 56–58 °C, $Kp_{12}$ 173–176 °C

2-[Indanyl-(5)-oxy]-ethylamin

109 g Indanyl-(5)-oxy-acetonitril werden in Gegenwart von Raney-Nickel in 700 ml Ethanol und 180 ml flüss. Ammoniak bei 110 at und 90 °C hydriert. Nach Destillation erhält man 86 g farbloses Öl, $Kp_{12}$ 154–156 °C.

Analog werden aus 2,3-Dimethylphenoxy-acetonitril bzw. 3,4-Methylendioxyphenoxy-acetonitril durch Hydrierung erhalten:
2-(2,3-Dimethylphenoxy)-ethylamin, $Kp_{12}$ 129–132 °C
2-(3,4-Methylendioxyphenoxy)-ethylamin, $Kp_{13}$ 162–164 °C

2-(2-Methylmercaptophenoxy)-ethylamin

26,7 g (2-Methylmercaptophenoxy)-acetonitril werden mit 8,5 g Lithiumaluminiumhydrid in 1,3 Liter Ether reduziert (4 Stunden Rückfluss). Nach üblicher Aufarbeitung und Destillation werden 21,0 g farbloses Öl, $Kp_{0,1}$ 117–120 °C, erhalten.

Analog durch Reduktion von 2,6-Dimethoxyphenoxy-acetonitril:

2-(2,6-Dimethoxyphenoxy)-ethylamin, $Kp_{12}$ 160–162 °C

Beispiel 4

1-[Carbazolyl-(4)-oxy]-3-[2-(2-methylphenoxy)-ethylamino]-propanol-(2)

6,0 g 4-(2,3-Epoxypropoxy)-carbazol und 7,6 g 2-(2-Methylphenoxy)-ethylamin werden 20 Stunden bei 70 °C gerührt. Man löst in Methylenchlorid und trennt das Gemisch durch Chromatographie an einer Kieselgelsäule (500 ml) mit den Laufmitteln Methylenchlorid, Methylenchlorid-Essigester (9:1 und 7:3), Essigester und Essigester-Methanol (9:1). Reihenfolge der Elution: Tertiäres Amin, sekundäres Amin, primäres Ausgangsamin. Nach Anreiben mit Ether und Umkristallisieren aus Essigester unter Verwendung von Aktivkohle und Bleicherde werden 5,2 g (53% d.Th.) farblose Kristalle, Fp. 125–126 °C, erhalten.

In analoger Weise erhält man:

a) 1-[Carbazolyl-(4)-oxy]-3-[2-(3-methylphenoxy)-ethylamino]-propanol-(2)
43% d.Th., Fp. 129–130 °C,
aus 4-(2,3-Epoxypropoxy)-carbazol und 2-(3-Methylphenoxy)-ethylamin.

b) 1-[Carbazolyl-(4)-oxy]-3-[2-(2-chlorphenoxy)-ethylamino]-propanol-(2)
26% d.Th., Fp. 111–112 °C,
aus 4-(2,3-Epoxypropoxy)-carbazol und 2-(2-Chlorphenoxy)-ethylamin.

c) 1-[Carbazolyl-(4)-oxy]-3-[2-(3-methoxyphenoxy)-ethylamino]-propanol-(2)
22% d.Th., Fp. 111–113 °C,
aus 4-(2,3-Epoxypropoxy)-carbazol und 2-(3-Methoxyphenoxy)-ethylamin.

d) 1-[Carbazolyl-(4)-oxy]-3-[2-(4-methoxyphenoxy)-ethylamino]-propanol-(2)
48% d.Th., Fp. 106–108 °C,
aus 4-(2,3-Epoxypropoxy)-carbazol und 2-(4-Methoxyphenoxy)-ethylamin.

e) 1-[Carbazolyl-(4)-oxy]-3-[2-(2-methoxyphenylmercapto)-ethylamino]-propanol-(2)
15% d.Th., Fp. 108–109 °C,
aus 4-(2,3-Epoxypropoxy)-carbazol und 2-(2-Methoxyphenylmercapto)-ethylamin.

f) 1-[Carbazolyl-(4)-oxy]-3-[1-(2-methoxyphenoxy)-propyl-(2)-amino]-propanol-(2)
85% d.Th., Fp. 112–125 °C (rohes Gemisch der Diastereomeren), daraus durch Umkristallisieren aus Ethanol, Essigester und Toluol-Isopropanol, farblose Kristalle, Fp. 140–141 °C und aus der Mutterlauge ein weiteres Produkt
Fp. 121,5-122,5 °C.
aus 4-(2,3-Epoxypropoxy)-carbazol und 1-(2-Methoxyphenoxy)-propyl-(2)-amin.

g) 1-[Carbazolyl-(4)-oxy]-3-[2-(2-methylsulfinylphenoxy)-ethylamino]-propanol-(2)
25% d.Th.; Oxalat ab 126 °C. Zers.
aus 4-(2,3-Epoxypropoxy)-carbazol und 2-(2-Methylsulfinylphenoxy)-ethylamin.

Die Verbindung wird auch erhalten durch Oxidation von 1-[Carbazolyl-(4)-oxy]-3-[2-(2-methylmercaptophenoxy)-ethylamino]-propanol-(2), vgl. Beispiel 3i, mit der äquivalenten Menge Wasserstoffperoxid in Essigsäure bei Raumtemperatur.

Die Ausgangsamine der Beispiele 4e, 4f und 4g können durch literaturanaloge Umsetzungen wie folgt hergestellt werden:

2-(2-Methoxyphenylmercapto)-ethylamin

Durch Umsetzung von o-(2-Chlorethylmercapto)-anisol in flüss. Ammoniak (8 Stunden 120 °C); Öl, $Kp_{0,05}$ 118–122 °C, Hydrochlorid Fp. 163–167 °C.

1-(2-Methoxyphenoxy)-propyl-(2)-amin

Durch Hydrierung von 2-Methoxyphenoxyaceton in Ammoniak-Ethanol (120 at, 90 °C); Öl, $Kp_{13}$ 144–146 °C, Oxalat Fp. 199–200 °C (Z.).

2-(2-Methylsulfinylphenoxy)-ethylamin

Durch Oxidation von 2-(2-Methylmercapto)-ethylamin mit 1 Äquivalent Perhydrol (30%) in Essigsäure bei Raumtemperatur; Öl, Oxalat Fp. 174–175 °C.

Beispiel 5

1-[Carbazolyl-(4)-oxy]-3-[N-benzyl-2-(2-methoxyphenoxy)-ethylamino]-propanol-(2)

15,1 g 4-(2,3-Epoxypropoxy)-carbazol und 16,2 g N-[2-(2-Methoxyphenoxy)-ethyl]-benzylamin in 50 ml Ethylenglykoldimethylether werden 24 Stunden unter Rückfluss erhitzt. Man bringt zur Trockene, reinigt über eine Kieselgelsäule mit den Laufmitteln Methylenchlorid, Methylenchlorid-Essigester (9:1 und 7:3), Essigester und reibt den Rückstand der Hauptfraktion mit Ether an. Ausbeute: 25,0 g (80% d.Th.) farblose Kristalle, Fp. 97–99 °C.

In analoger Weise erhält man:

a) 1-[Carbazolyl-(4)-oxy]-3-[N-methyl-2-(2-methoxyphenoxy)-ethylamino]-propanol-(2)
22% d.Th., farbloses Öl, Hydrochlorid Fp. 109 °C (leichtes Gasen),
aus 4-(2,3-Epoxypropoxy)-carbazol und N-Methyl-2-(2-methoxyphenoxy)-ethylamin.

b) 1-[Carbazolyl-(4)-oxy]-3-[N-butyl-2-(2-methoxyphenoxy)-ethylamino]-propanol-(2)
84% d.Th., farbloses Öl, Hydrochlorid Fp. 169–170 °C,
aus 4-(2,3-Epoxypropoxy)-carbazol und N-[2-(2-Methoxyphenoxy)-ethyl]-butylamin.

c) 1-[Carbazolyl-(4)-oxy]-3-[N-benzyl-2-(5-carbamoyl-2-pyridyloxy)-ethylamino]-propanol-(2)
80% d.Th., Fp. 165–167 °C,
aus 4-(2,3-Epoxypropoxy)-carbazol und N-[2-(5-Carbamoyl-2-pyridyloxy)-ethyl]-benzylamin.

Beispiel 6

1-[Carbazolyl-(4)-oxy]-2-formyloxy-3-[N-benzyl-2-(2-methoxyphenoxy)-ethylamino]-propan-hydrochlorid

Man lässt auf 7,9 g 1-[Carbazolyl-(4)-oxy]-3-[N-benzyl-2-(2-methoxyphenoxy)-ethylamino]-propanol-(2) ein aus 3 ml Ameisensäure und 6 ml Essigsäureanhydrid bereitetes Ameisensäure-Essigsäure-Anhydridgemisch während 2½ Tagen bei Raumtemperatur einwirken, giesst in Eiswasser, neutralisiert mit Natriumhydrogencarbonatlösung, extrahiert mit Methylenchlorid und fällt aus einer etherischen Lösung des Extraktrückstandes das Hydrochlorid. Ausbeute: 8,1 g (91% d.Th.) farblose Kristalle, ab 85 °C Sintern, ab 120 °C Blasenbildung.

Beispiel 7

1-[Carbazolyl-(4)-oxy]-2-pivaloyloxy-3-[N-benzyl-2-(2-methoxyphenoxy)-ethylamino]-propan-

hydrochlorid

Zu einer Lösung von 7,0 g 1-[Carbazolyl-(4)-oxy]-3-[N-benzyl-2-(2-methoxyphenoxy)-ethylamino]-propanol-(2) in 35 ml Pyridin wird 1,9 ml Pivalinsäurechlorid eingetragen. Nach Stehen über Nacht giesst man in Wasser, nimmt in Methylenchlorid auf, reinigt chromatographisch über eine Kieselgelsäule und fällt aus einer etherischen Lösung der Base das Hydrochlorid.

Ausbeute: 6,6 g (7% d.Th.) farblose Kristalle, ab 102°C Sintern, Fp. 120°C (leichtes Gasen).

In analoger Weise erhält man durch Benzoylierung:

1-[Carbazolyl-(4)-oxy]-2-benzoyloxy-3-[N-benzyl-2-(2-methoxyphenoxy)-ethylamino]-propan-hydrochlorid

70% d.Th., Fp. 113°C (leichtes Gasen)

Beispiel 8

1-[Carbazolyl-(4)-oxy]-2-formyloxy-3-[2-(2-methoxyphenoxy)-ethylamino]-propan-hydrochlorid

2,2 g 1-[Carbazolyl-(4)-oxy]-2-formyloxy-3-[N-benzyl-2-(2-methoxyphenoxy)-ethylamino]-propan-hydrochlorid werden in 40 ml abs. Tetrahydrofuran in Gegenwart von 0,3 g 10-proz. Palladium-Kohle bei Normaldruck hydriert. Der nach Absaugen und Einengen erhaltene Rückstand wird beim Durcharbeiten mit Ether kristallin.

Ausbeute: 1,3 g (70% d.Th.) farblose Kristalle, Fp. 62°C (unter Blasenbildung).

In analoger Weise erhält man:

a) 1-[Carbazolyl-(4)-oxy]-2-pivaloyloxy-3-[2-(2-methoxyphenoxy)-ethylamino]-propan-hydrochlorid

85% d.Th., Fp. 199–201°C (leichtes Gasen), durch Hydrogenolyse von 1-[Carbazolyl-(4)-oxy]-2-pivaloyloxy-3-[N-benzyl-2-(2-methoxyphenoxy)-ethylamino]-propan-hydrochlorid.

b) 1-[Carbazolyl-(4)-oxy]-2-benzoyloxy-3-[2-(2-methoxyphenoxy)-ethylamino]-propan-hydrochlorid

84% d.Th., Fp. 102°C (unter Gasen), durch Hydrogenolyse von 1-[Carbazolyl-(4)-oxy]-2-benzoyloxy-3-[N-benzyl-2-(2-methoxyphenoxy)-ethyalmino]-propan-hydrochlorid.

c) 1-[Carbazolyl-(4)-oxy]-3-[2-(5-carbamoyl-2-pyridyloxy)-ethylamino]-propanol-(2)

Fp. 176–178°C, durch Hydrogenolyse von 1-[Carbazolyl-(4)-oxy]-3-[N-benzyl-2-(5-carbamoyl-2-pyridyloxy)-ethylamino]-propanol-(2).

d) 1-[Carbazolyl-(4)-oxy]-3-[2-(2-hydroxyphenoxy)-ethylamino]-propanol-(2)

77% d.Th., Hydrochlorid, Fp. 214–215°C, durch Hydrogenolyse von 1-[Carbazolyl-(4)-oxy]-3-[2-(2-benzyloxyphenoxy)-ethylamino]-propanol-(2).

Beispiel 9

1-[Carbazolyl-(4)-oxy]-3-[2-(5-fluor-2-methoxyphenoxy)-ethylamino]-propanol-(2)

7,0 g 1-Amino-3-[carbazolyl-(4)-oxy]-propanol-(2), 9,2 g p-Toluolsulfonsäure-[2-(5-fluor-2-methoxyphenoxy)-ethylester] und 3,8 ml Triethylamin werden in 20 ml Dimethylformamid 20 Stunden bei 70°C gerührt. Man giesst in verdünnte Natronlauge, extrahiert mit Methylenchlorid, trocknet und reinigt chromatographisch wie in Beispiel 4 angegeben. Nach Umkristallisieren aus Essigester unter Verwendung von Aktivkohle und Bleicherde werden 2,7 g (23% d.Th.) farblose Kristalle, Fp. 146–147°C, erhalten.

Die Ausgangsverbindungen können wie folgt hergestellt werden:

1-Amino-3-[carbazolyl-(4)-oxy]-propanol-(2)

40 g 4-(2,3-Epoxypropoxy)-carbazol werden mit 500 ml flüss. Ammoniak in 2 Liter Methanol 24 Stunden bei 50°C gerührt (Autoklav). Nach Einengen und Umkristallisieren aus Ethanol: 31 g farblose Kristalle, Fp. 141–143°C.

p-Toluolsulfonsäure-[2-(5-fluor-2-methoxyphenoxy)-ethylester]

40,4 g 5-Fluor-2-methoxyphenol, 24,6 ml 2-Chlorethanol und 20,7 g Kaliumhydroxid werden in 100 ml DMF 2 Stunden bei 70°C gerührt. Man giesst in Wasser, extrahiert mit Methylenchlorid, destilliert den Extraktrückstand im Feinvakuum und erhält 11,3 g 2-(5-Fluor-2-methoxyphenoxy)-ethanol, farbloses Öl, welches beim Stehen erstarrt, Fp. 43–45°C. Die weitere Umsetzung mit p-Toluolsulfonsäurechlorid ergibt das Tosylat Fp. 66–68°C (aus Ethanol).

Beispiel 10

1-[Carbazolyl-(4)-oxy]-3-[1-(2-methoxyphenoxy)-propyl-(2)-amino]-propanol-(2)

Man hydriert ein Gemisch aus 8,1 g 1-Amino-3-[carbazolyl-(4)-oxy]-propanol-(2) und 6,0 g (2-Methoxyphenoxy)-aceton in 250 ml Methanol in Gegenwart von 1,0 g 10-proz. Palladiumkohle (5 at, 38°C) und reinigt das erhaltene Rohprodukt chromatographisch wie in Beispiel 4 angegeben. Nach Anreiben des Rückstandes der Hauptfraktion werden 5,5 g (41% d.Th.) farblose Kristalle, Fp. 113–117°C, rohes Diastereomerengemisch erhalten. Durch Umkristallisieren aus Essigester und aus Ethanol wird daraus ein Produkt mit konstantem Fp. 140–141°C erhalten.

Beispiel 11

1-[Carbazolyl-(4)-oxy]-3-[3-(2-methoxyphenyl)-propylamino]-propanol-(2)

Zu einer Lösung von 6,0 g 1-Amino-3-[carbazolyl-(4)-oxy]-propanol-(2) und 3,3 ml Triethylamin in 50 ml Methylenchlorid wird bei Raumtemperatur eine Lösung von 4,4 g 3-(2-Methoxyphenyl)-propionsäurechlorid in 50 ml Methylenchlorid unter Rühren zugetropft. Nach Stehen über Nacht schüttelt man mit Wasser aus, trocknet die organische Phase, engt ein, rührt den Rückstand mit Ether aus und erhält 8,2 g (84% d.Th.) 1-[Carbazolyl-(4)-oxy]-3-[3-(2-methoxyphenyl)-propionylamino]-propanol-(2), Fp. 142–144°C. 7,7 g dieses Zwischenproduktes werden mit 1,5 g Lithiumaluminiumhydrid in 100 ml abs. Tetrahydrofuran reduziert (20 Stunden Rückfluss). Das nach üblicher Aufarbeitung erhaltene Öl wird chromatographisch über eine Kieselgelsäule gereinigt (vgl. Beispiel 4). Durch Umkristallisieren aus Toluol unter Verwendung von Aktivkohle und Bleicherde werden 2,1 g (28% d.Th.) farblose Kristalle, Fp. 102–104 °C, erhalten.

**Patentansprüche**

1. Carbazolyl-(4)-oxy-propanolamin-Derivate der Formel I

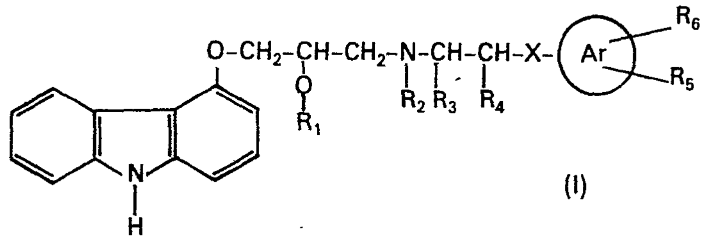

(I)

in der

$R_1$ Wasserstoff, eine niedere Alkanoylgruppe oder eine Benzoyl- oder Naphthoylgruppe,

$R_2$ Wasserstoff, eine niedere Alkylgruppe oder eine Benzyl-, Phenylethyl- oder Phenylpropylgruppe,

$R_3$ Wasserstoff oder eine niedere Alkylgruppe,

$R_4$ Wasserstoff oder eine niedere Alkylgruppe,

X einen Valenzstrich, eine $-CH_2$-Gruppe, ein Sauerstoff- oder Schwefelatom,

Ar einen Phenyl-, Naphthyl-, Indanyl-, Tetrahydronaphthyl- oder Pyridyl-Rest und

$R_5$ und $R_6$, welche gleich oder verschieden sein können, jeweils Wasserstoff, Halogen, eine niedere Alkylgruppe, eine Aminocarbonylgruppe, eine Hydroxygruppe, eine niedere Alkoxygruppe, eine Benzyloxygruppe, eine niedere Alkylmercaptogruppe, eine niedere Alkylsulfinylgruppe, eine niedere Alkylsulfonylgruppe oder auch gemeinsam eine Methylendioxygruppe bedeuten, wobei für den Fall, dass X ein Sauerstoffatom darstellt, $R_4$ und $R_5$ gemeinsam auch die Gruppe $-CH_2-O-$ bedeuten kann,

sowie deren physiologisch verträglichen Salze.

2. Verfahren zur Herstellung von Carbazolyl-(4)-oxy-propanolamin-Derivaten gemäss Anspruch 1 mit den dort angegebenen Bedeutungen für $R_1$, $R_2$, $R_3$, $R_4$, X, Ar, $R_5$ und $R_6$ sowie deren physiologisch verträglichen Salzen, dadurch gekennzeichnet, dass man nach an sich bekannter Weise entweder

a) eine Verbindung der Formel II

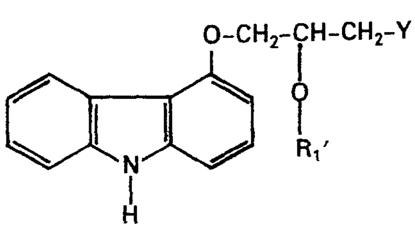

(II)

in welcher Y eine reaktive Gruppe darstellt und $R_1'$ die für $R_1$ angegebene Bedeutung hat oder Y und $R_1'$ zusammen einen Valenzstrich bedeuten, mit einer Verbindung der Formel III

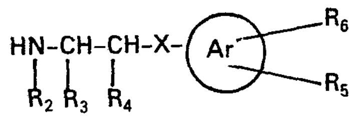

(III)

in welcher $R_2$, $R_3$, $R_4$, X, Ar, $R_5$ und $R_6$ die angegebene Bedeutung haben,
umsetzt oder

b) eine Verbindung der Formel IV

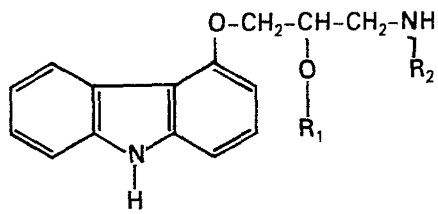

(IV)

in welcher $R_1$ und $R_2$ die angegebene Bedeutung haben, mit einer Verbindung der Formel V

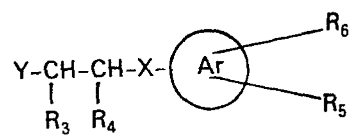

(V)

in welcher Y, $R_3$, $R_4$, X, Ar, $R_5$ und $R_6$ die angegebene Bedeutung haben,
umsetzt oder

c) ein Gemisch aus einer Verbindung der Formel IV und einer Verbindung der Formel VI

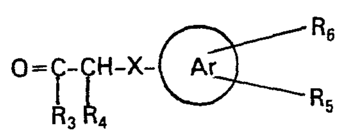

(VI)

in welcher $R_3$, $R_4$, X, Ar, $R_5$ und $R_6$ die angegebene Bedeutung haben,
reduziert oder

d) eine Verbindung der Formel IV mit einer Verbindung der Formel VII

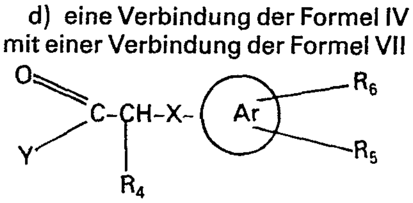

(VII)

in welcher Y, $R_4$, X, Ar, $R_5$ und $R_6$ die angegebene Bedeutung haben,
umsetzt und das so erhaltene Amid reduziert, worauf man gegebenenfalls die nach obigen Verfahren hergestellten Verbindungen der allgemeinen Formel I in andere Verbindungen der Formel I umwandelt sowie gewünschtenfalls die erhaltenen Verbindungen der Formel I in ihre pharmakologisch verträglichen Salze überführt.

3. Verwendung von Verbindungen der Formel I und deren pharmakologisch verträglichen Salzen zur Bereitung von Arzneimitteln mit vasodilatierender und/oder β-rezeptorenblockierender Wirkung.

4. Arzneimittel enthaltend einen Wirkstoff der Formel I sowie an sich bekannte pharmakologisch verträgliche Trägerstoffe.

**Claims**

1. Carbazolyl-(4)-oxypropanolamine derivatives of the formula I

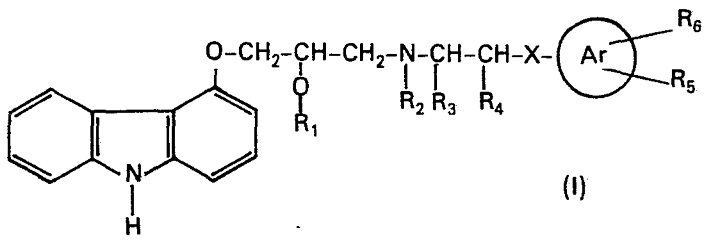

in which $R_1$ signifies hydrogen, a lower alkanoyl group or a benzoyl or naphthoyl group,
$R_2$ hydrogen, a lower alkyl group or a benzyl, phenylethyl or phenylpropyl group,
$R_3$ hydrogen or a lower alkyl group,
$R_4$ hydrogen or a lower alkyl group,
X is a valency bond, a $-CH_2-$ group, an oxygen or sulphur atom,
Ar a phenyl, naphthyl, indanyl, tetrahydronaphthyl or a pyridyl radical and
$R_5$ and $R_6$, which can be the same or different, each hydrogen, halogen, a lower alkyl group, an aminocarbonyl group, a hydroxyl group, a lower alkoxy group, a benzyloxy group, a lower alkylmercapto group, a lower alkylsulphinyl group, a lower alkylsulphonyl group or also together a methylenedioxy group, whereby, for the case in which X represents an oxygen atom, $R_4$ and $R_5$ together can also signify the group $-CH_2-O-$, as well as their physiologically acceptable salts.

2. Process for the preparation of carbazolyl-(4)-oxypropanolamine derivatives according to claim 1 with the there-given meanings for $R_1$, $R_2$, $R_3$, $R_4$, X, Ar, $R_5$ and $R_6$, as well as of their physiologically acceptable salts, characterised in that, in per se known manner, one either

a) reacts a compound of the formula II

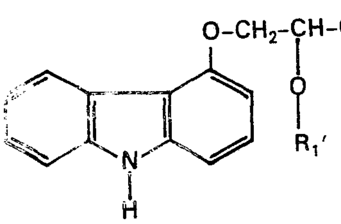

(II)

in which Y represents a reactive group and $R_1'$ has the meaning given for $R_1$ or Y and $R_1'$ together represent a valency bond, with a compound of the formula III

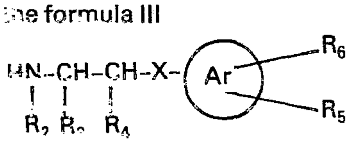

(III)

in which $R_2$, $R_3$, $R_4$, X, Ar, $R_5$ and $R_6$ have the given meaning; or

b) reacts a compound of the formula IV

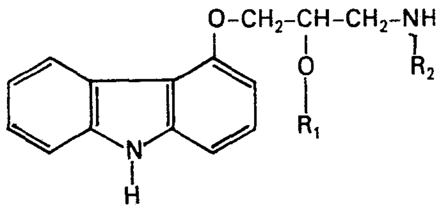

(IV)

in which $R_1$ and $R_2$ have the given meaning, with a compound of the formula V

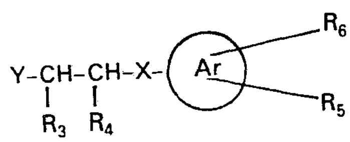

(V)

in which Y, $R_3$, $R_4$, X, Ar, $R_5$ and $R_6$ have the given meaning; or

c) reduces a mixture of a compound of the formula IV and of a compound of the formula VI

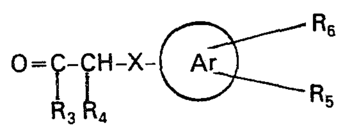

(VI)

in which $R_3$, $R_4$, X, Ar, $R_5$ and $R_6$ have the given meaning; or

d) reacts a compound of the formula IV with a compound of the formula VII

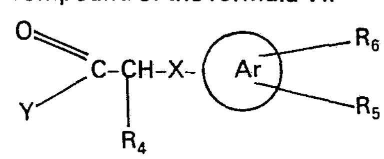

(VII)

in which Y, $R_4$, X, Ar, $R_5$ and $R_6$ have the given meaning, and reduces the so obtained amide, whereafter one possibly converts the compounds

of general formula I prepared according to the above process into other compounds of formula I, as well as, if desired, converts the compounds obtained of formula I into their pharmacologically acceptable salts.

3. Use of compounds of formula I and of their pharmacologically acceptable salts for the preparation of medicaments with vasodilatory and/or β-receptor-blocking action.

4. Medicaments containing an active material of formula I, as well as per se known pharmacologically compatible carrier materials.

**Revendications**

1. Dérivés de carbazolyl-(4)-oxy-propanolamine de formule I

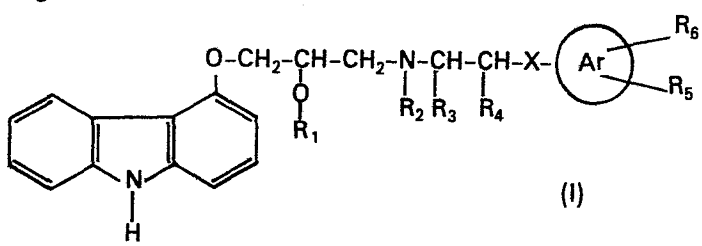

(I)

dans laquelle:
$R_1$ est un atome d'hydrogène, un groupe alcanoyle inférieur ou un groupe benzoyle ou naphtoyle,
$R_2$ est un atome d'hydrogène, un groupe alkyle inférieur, ou un groupe benzyle, phényléthyle ou phénylpropyle,
$R_3$ est de l'hydrogène ou un groupe alkyle inférieur,
$R_4$ est de l'hydrogène ou un groupe alkyle inférieur,
X est un trait de valence, un groupe $-CH_2-$, un atome d'oxygène ou un atome de soufre,
Ar est un reste phényle, naphtyle, indanyle, tétrahydronaphtyle ou pyridyle et
$R_5$ et $R_6$, pouvant être identiques ou différents sont chacun de l'hydrogène, du chlore, un groupe alkyle inférieur, un groupe amino carbonyle, une fonction hydroxyle, un groupe alcoxyle inférieur, un groupe benzyloxy, un groupe alkyl-mercapto inférieur, un groupe alkylsulfinyle inférieur, un groupe alkylsulfonyle inférieur ou bien ils forment ensemble un groupe méthylènedioxy, et dans le cas où
X est un atome d'oxygène, $R_4$ et $R_5$ peuvent aussi désigner ensemble le groupe $-CH_2O-$,
ainsi que leurs sels physiologiquement tolérables.

2. Procédé pour la préparation des dérivés de carbazolyl-(4)-oxy-propanolamine selon la revendication 1 dans lesquels $R_1$, $R_2$, $R_3$, $R_4$, X, Ar, $R_5$ et $R_6$ ont la signification donnée ci-dessus, ainsi que leurs sels physiologiquement tolérables, caractérisé en ce que l'on fait réagir de façon en soi connue:

a) un composé de formule II

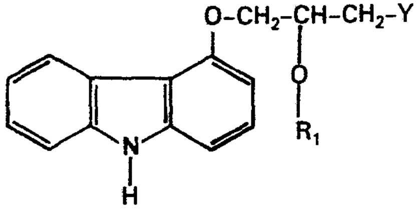

(II)

dans laquelle Y est un groupe réactif et $R_1'$ a la signification donnée pour $R_1$ ou bien Y et $R_1'$ forment ensemble un trait de valence,
avec un composé de formule III

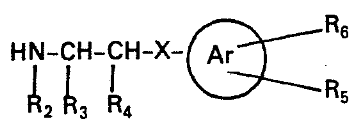

(III)

dans laquelle $R_2$, $R_3$, $R_4$, X, Ar, $R_5$ et $R_6$ ont la signification indiquée, ou,

b) un composé de formule IV

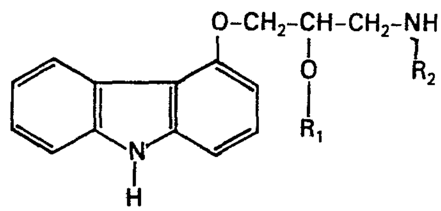

(IV)

dans laquelle $R_1$ et $R_2$ ont la signification indiquée, avec un composé de formule V

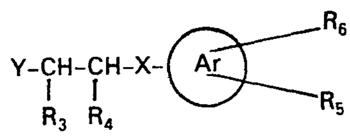

(V)

dans laquelle Y, $R_3$, $R_4$, X, Ar, $R_5$ et $R_6$ ont la signification indiquée, ou,

c) un mélange d'un composé de formule IV et un composé de formule VI

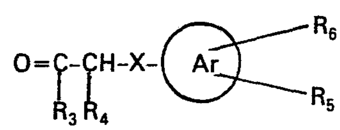

(VI)

dans laquelle $R_3$, $R_4$, X, Ar, $R_5$ et $R_6$ ont la signification indiquée, en le soumettant à une réduction, ou,

d) un composé de formule IV avec un composé de formule VII

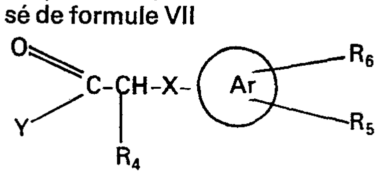

(VII)

dans laquelle Y, $R_4$, X, Ar, $R_5$ et $R_6$ ont la signification indiquée, et on soumet l'amide obtenu à une réduction, et en ce qu'ensuite on transforme éventuellement les composés de formule I obtenus selon le procédé ci-dessus en d'autres composés de formule I, et les composés de formule I obtenus en leurs sels pharmacologiquement tolérables.

3. Utilisation des composés de formule I et de leurs sels pharmacologiquement tolérables pour la préparation de médicaments ayant une activité vasodilatrice et/ou une activité de blocage des β-récepteurs.

4. Médicaments comprenant une substance active de formule I ainsi que des substances de support pharmacologiquement tolérables.